Europäisches Patentamt

**European Patent Office**

**Office européen des brevets**

(19)

(11) Publication number: **0 213 935**
**A2**

(12) # EUROPEAN PATENT APPLICATION

(21) Application number: **86306654.4**

(22) Date of filing: **28.08.86**

(51) Int. Cl.⁴: **A 61 K 31/395**
**C 07 D 498/08**
**//(C07D498/08, 307:00, 267:00)**

(30) Priority: **29.08.85 US 770807**

(43) Date of publication of application:
**11.03.87 Bulletin 87/11**

(84) Designated Contracting States:
**AT BE CH DE FR IT LI LU NL SE**

(71) Applicant: **AMERICAN HOME PRODUCTS CORPORATION**
**685, Third Avenue**
**New York, New York 10017(US)**

(72) Inventor: **Hung, Paul Porwen**
**506 Ramblewood Drive**
**Bryn Mawr Pennsylvania(US)**

(74) Representative: **Brown, Keith John Symons et al,**
**c/o John Wyeth & Brother Limited Patent and Trademark Department Huntercombe Lane South Taplow Maidenhead Berkshire SL6 0PH.(GB)**

(54) Rifamycin derivatives for treating hepatitis.

(57) The invention disclosed herein relates to a method of treating an animal infected by hepatitis B virus by administering to such infected animal an amount of a rifamycin derivative selected from a group consisting of

(a) 3-amino-4-deoxo-4-imino-rifamycin S,

(b) 3-amino-4-deoxo-4- [(4-aminophenyl) sulphonyl] -amino-rifamycin SV,

(c) 4-O-n-butylsulphonyl-3- [(1-piperidinylimino)methyl] -rifamycin SV,

(d) 4-O-n-butylsulphonyl-3- [(4-morpholinylimino) methyl] rifamycin SV,

(e) 3-[(dimethylhydrazono) methyl]-4-O-[(3-phenylpropyl) sulphonyl]-rifamycin SV and

(f) 4-deoxy-N,3-ethylidenimino-4- (methylsulphonyl)- amino- rifamycin SV

said amount being effective to alleviate the effects of said hepatitis B virus. The invention particularly relates to the use of the above mentioned rifamycin derivatives for the manufacture of a medicament for treating or preventing hepatitis. Also disclosed is a method of inhibiting the viral polymerase of hepatitis B virus by subjecting said polymerase to an amount of one of the rifamycin derivatives (a) - (f) above, said amount being effective to prevent the synthesis of hepatitis B virus by said viral polymerase. Compounds (b) to (f) described above are novel compounds.

Hepatitis caused by hepatitis B virus (HBV) is one of the last few major diseases which has not been effectively controlled. No effective treatment or preventative agent has yet been found for this disease. Applicant's invention pertains to a method of treating and preventing hepatitis caused by hepatitis B virus utilising certain rifamycin S and SV derivatives.

Hepatitis B virus (HBV) is prevalent throughout the world, but is especially endemic to parts of Asia and Africa. Acute infection is usually resolved after 4-6 months; however, chronic infection often lasts a lifetime and is highly associated, inter alia, with the development of cirrhosis and primary hepatocellular carcinoma. See, for example, Beasley and Hwang, , "Epidimiology of Hepatocellular Carcinoma," Chapter 16, pp. 209-224 in Vyas, Girish N. et al., eds., Viral Hepatitis and Liver Disease, (Grune and Stratton, Inc., Orlando, Florida, 1984), where it is reported that, on a worldwide basis, 80 percent of the people in whom primary hepatocellular carcinoma occurs are hepatitis B surface antigen (HBsAg) carriers. The 200 million chronic HBV carriers worldwide can act as a reservoir of infectious HBV in the general population. Accordingly, the development of an effective treatment of hepatitis B viral infections, especially of chronic hepatitis, is urgently needed to arrest the increasing spread of this disease.

Chronic HBV infection can be categorized into chronic active hepatitis (CAH) and chronic persistent hepatitis. CAH symptoms encompass fatigue, nausea, anorexia and/or abdominal pain, and general depression of liver function. Chronic persistent HBV is associated with cirrhosis of the liver. As noted above acute HBV infection is usually resolved in 4-6

0213935

- 3 -

months. Its symtoms encompass fever, malaise, fatigue, anorexia, nausea, vomiting and abdominal pain. A small portion of acute HBV infections result in massive hepatic necrosis, coma and death. No chemotherapeutic agent has yet been found to be effective for treatment of acute viral hepatitis (Gitlin in Vyas, Girish N. et al., eds., above, Chapter 8,pp. 115-122, at page 115).

Ideally antiviral agents for HBV infection could be used to eliminate HBV infection from chronic carriers, thus decreasing their chances of developing HBV associated cirrhosis and primary heptocellular carcinoma. In addition, antviral agents could be used to speed up recovery from acute HBV infection. At present no such chemotherapeutic agent has been identified. The development of such antiviral agents has been hindered by three major factors. First, the host range of HBV appears to be confined to humans, the natural host, and a few higher primates such as chimpanzees. Although three related viruses which infect the Beechey ground squirrel (Ground Squirrel Hepatitis Virus GSHV), the woodchuck (Woodchuck Hepatitis Virus: WVH) and the domestic duck L (Duck Hepatitis B Virus: DHBV), have been found, none of these viruses can infect nor replicate in common laboratory animals such as rats and mice (Summers, Hepatology 1 : 179-183, 1980). Secondly, no laboratories have been able to infect and propogate HBV in tissue culture. Recently, Tuttleman et al (Molecular Biology of Hepatitis B Viruses, Cold Spring Harbor Labs, pg. 9, 1985) have described a tissue culture system in which 5% primary duck hepatocytes can be infected with the duck hepatitis B virus (DHBV), a member of the hepatitis B virus family. However, since the protocol utilizes normal hepatocytes from young ducklings, the system cannot be readily adopted for propogation of HBV in human hepatocytes (i.e. lack of

- 4 -

donor hepatocytes). Thirdly, the study of the mechanism of replication of HBV, characterization of the viral gene Products, and elucidation of factors which influance the outcome of the infection has progressed slowly due to the aforementioned factors.

In spite of these obstacles, there have been attempts at and partial success in providing a method of treatment of hepatitis B virus by chemotherapy and/or immunotherapy. However, a practical, effective treatment for hepatitis B virus infection has not been found. One such attempt and partial success is described by C.I. Smith and William S. Robinson, et al., "Acute Dane Particle Suppression with Recomninant Leukocyte A Interferon in Chronic Hepatitis B Virus Infection," The Journal of Infectious Diseases, 148, 907-13 (1983). In this method Robinson and his coworkers used a leukocyte A Interfereon (rINF-A or HuIFN-2), produced by recombinant DNA methods, to treat nine patients with chronic hepatitis B virus infection. They report that most courses of rINF-A treatment were associated with a reduction in polymerase activity of the viron or Dane particle. However, this change was not permanent in any of the patients.

One means of developing an effective treatment for HBV infection is to invent a compound which inhibits the function of a particular viral protein which is necessary for viral replication, such as a viral-associated polymerase. Robinson and colleagues discovered a polymerase inside the HBV particles isolated from serum (Kaplan, et al., J. Virol 12: 995-1005, 1973). Its presence can be readily detected by the endogenous polymerase assay which measures the ability of the polymerase to incorporate radiolabeled nucleotides into the viral genome. Further studies showed that only one strand, called the short strand or + strand was being

- 5 -

synthesized in each particle (Summers et al. Proc. Nat'l.
Acad. Science 72,4597-4601, 1975; Landers et al., J.
Virol. 23, 368-376,1977). Using the related duck
hepatitis B virus animal model, Summers and Mason (Cell 29,
403-415,1982) have isolated immature viral particles
from infected duck hepatocytes and showed that (1) the
RNA pregenome was packaged in immature particles, (2) an
endogenous polymerase synthesized the minus strand DNA
from the RNA template by reverse transcription (3) the
RNA template was degraded by an RNAse H activity present in
the particles, (4) the plus strand was synthesized using
the minus (-) strand DNA as a template, and (5) the
polymerase associated with the immature viral particles
did exhibit reverse transcriptase-like activity. Recent
studies of HBV, GSHV, and WHV DNA intermediates isolated
from infected liver revealed asymmetric synthesis of
minus strand, consistent with the above findings (Weiser
et al., J. Virol. 48, 1-9, 1983; Fowler et al., J. Med.
Virol. 13, 83-91, 1984; Roggendorf and Summers, Molecular
Biology of Hepatitis B Viruses, Cold Spring Harbor
Laboratories, pg. 5, 1985). Although no laboratory has
directly shown that the polymerase found in the viral
particles is encoded by the virus itself, Toh et al
(Nature 305, 827-829, 1983) have reported the existence of
extensive amino acid sequence homology in specific regions
of a large open reading frame encoded by HBV (adr strain)
and WHV and three other retroviral reverse transcriptases.
This analysis lends some credence to the hypothesis that
the reverse transcriptase associated with viral particles
is encoded by the viral genome.

Since the HBV polymerase exhibits reverse
transcriptase properties, one means of arriving at a
method of treatment would be to test known inhibitors
of similar reverse transcriptases, for example retroviral
reverse transcriptases. Some rifamycin derivatives have been found to inhibit particular reverse transcriptases.
The following articles disclose certain rifamycin
derivatives which inhibit particular reverse transcriptase
(polymerases): Maurice Green et al., "3 Cyclic Amine
Derivatives of Rifamycin: Strong Inhibitors of the DNA
Polymerase Activity of RNA Tumor Viruses," Proceedings
of the National Academy of Sciences (P.N.A.S.),69, 1294-98
(1972); Frances M. Thompson et al., "Inhibition of Three
Nucletide Polymerases by Rifamycin Derivatives," P.N.A.S.
71, 107-9 (1974); Alan M. Wu, et al., "RNA Directed DNA
Polymerase and Virus-Induced Leukemia in Mice," P.N.A.S.70,
1298-1302 (1973); and Corrado Gurgo et al., "Rifamycin
Derivatives strongly Inhibiting RNA DNA Polymerase
(Reverse Transcriptase) of Murine Sarcoma Viruses,"
Journal of the National Cancer Institute, 49, 61-79(1972).

Applicant herein has now surprisingly  found
a few rifamycin derivatives which inhibit the polymerase
activity of hepatitis B virus.    This provides.
means of treating hepatitis brought on by infection with
hepatitis B virus.

In one aspect of applicant's invention there is
provided a method of treating an animal infected by
hepatitis B virus comprising administering to such
infected animal an amount of a rifamycin derivative
selected from a group consisting of

    (a)   3-amino-4-deoxo-4-imino-rifamycin S

    (b)   3-amino-4-deoxo-4-[(4-aminophenyl)sulphonyl]
amino-rifamycin SV,

    (c)   4-O-n-butylsulphonyl-3-[(1-piperidinylimino)
methyl]-rifamycin SV,

    (d)   4-O-n-butylsulphonyl-3-[(4-morpholinylimino)
methyl]-rifamycin SV, and

    (e)   3-[(dimethylhydrazono)methyl]-4-O-[(3-
phenylpropyl)sulphonyl]-rifamycin SV and

(f) 4-deoxy-N,3-ethylidenimino-4-(methylsulphonyl)-
amino-rifamycin SV

said amount being effective to alleviate the effects of
said hepatitis B virus. With regard to this aspect of the
invention, the rifamycin derivatives (e) and (f) namely 3-
[(dimethylhydrazono)methyl]-4-O-[(3-phenylpropyl)sulphonyl]
rifamycin SV and 4-deoxy-N,3-ethylidenimino-4-(methylsulphonyl)amino-rifamycin SV are particularly preferred.
Also preferred is an amount of such rifamycin derivative,
i.e., (a) - (f), which is effective to prevent the reverse
transcription of the hepatitis B virus in the infected
animal. A further preferred amount of rifamycin derivative
(a) - (f) is an amount effective to show an absence of
HBeAg in standard serological tests in an animal (e.g.
man) previously found to be HBeAg positive. Similarly,
an amount effective to show the absence of HBV-DNA or DNA
polymerase where the same were present previously is also
preferred.

As noted above, hepatitis B virus is presently known
only to exist in man and in certain other primates and in
certain animals. However, should the virus be found in
other animals, some of which may act only as carriers of
the disease, then the method of treatment of the invention
would be applicable to such other animals also. Presently,
however, "infected animal" for Applicant's method of
treatment applies to man and those animals known to be
subject to the hepatitis B virus. The preferred animal
for such treatment is man.

In a further aspect this invention provides a method
of inhibiting the viral polymerase of hepatitis B virus,
comprising subjecting said polymerase to an amount of a
rifamycin derivative selected from a group consisting of

(a) 3-amino-4-deoxo-4-imino-rifamycin S,

(b) 3-amino-4-deoxo-4-[(4-aminophenyl)sulphonyl]
amino-rifamycin SV,

(c) 4-O-(n-butylsulphonyl)-3-[(1-piperidinylimino)
methyl-rifamycin SV,

(d) 4-O-(n-butylsulphonyl)-3-[(4-morpholinylimino)

methyl]-rifamycin SV,

(e)    3-[(dimethylhydrazono)methyl]-4-O-[(3-phenylpropyl)sulphonyl]-rifamycin SV and

(f)    4-deoxy-N,3-ethylidenimino-4-(methylsulphonyl)-amino-rifamycin SV

said amount being effective to prevent the synthesis of the hepatitis B virus by said polymerase. The preferred rifamycin derivatives for this aspect of the invention are also (e) and (f) named above. This amount may be shown by a reduction in the HBV-DNA titre or DNA polymerase activity titre in standard serological tests for the same.

In another aspect the invention provides the use of a rifamycin derivative selected from a group consisting of

(a)    3-amino-4-deoxo-4-imino-rifamycin S,

(b)    3-amino-4-deoxo-4-[(4-aminophenyl)sulphonyl] amino-rifamycin SV,

(c)    4-O-n-butylsulphonyl-3-[(1-piperidinylimino) methyl]-rifamycin SV,

(d)    4-O-n-butylsulphonyl-3-[(4-morpholinylimino) methyl]-rifamycin SV,

(e)    3-[(dimethylhydrazono)methyl]-4-O-[3-phenylpropyl-sulphonyl]-rifamycin SV and

(f)    4-deoxy-N,3-ethylidenimino-4-(methylsulphonyl)-amino-rifamycin SV

for the manufacture of a medicament for the treatment of an animal infected by hepatitis B virus.

In another aspect, this invention provides novel compounds (b), (c), (d), (e) and (f) described above. Surprisingly we have found that these novel compounds inhibit the polymerase of hepatitis B virus according to the procedure described below, while related rifamycin derivatives do not show such inhibition. Compounds (e) and (f) are also preferred for this aspect of the invention.

3-Amino-4-deoxo-4-imino-rifamycin S, (a) above, is described in U.S. Patent no. 4,017,481, where it is made by reacting 3-amino-rifamycin S with gaseous ammonia. The reaction may be carried out in a solution of tetrahydrofuran at room temperature for 15 hours with further addition of ammonia. The starting material, 3-amino-rifamycin S may be obtained as described in U.S. Patent no. 4,007,169, where it is obtained by reacting sodium azide with rifamycin S. The reaction may be carried out in methylformamide at 35°C for 60 minutes. The reaction mixture may be diluted with methylene chloride and washed with water. The aqueous phase contains rifamycin SV which may be oxidized by, for example, nitrous acid to the starting material rifamycin S. The organic phase may be washed with water, dried with sodium sulphate and evaporated to yield 3-amino rifamycin S which is recrystallized from 2-methoxy ethanol.

3-Amino-4-deoxo-4-[(4-aminophenyl)sulphonyl]amino-rifamycin SV, (b) above, may be made by reacting sodium p-analinylsulphinate with 3-amino-rifamycin S. The reaction may be carried out under neutral conditions in a solution solvent, such as dioxan at room temperature for up to 20 hours. The reaction may be carried out as described in Japanese Kokai J5 6053-682 (Derwent Abstract 46957 D/26).

Compound (f) may be made in known manner, e.g. by reacting acetaldehyde with 3-amino-4-deoxy-4-(methylsulphonyl)amino-rifamycin SV. The latter compound may be made in a similar manner to compound (b) but substituting sodium methanesulphonate for 4-aminobenzenesulphonate. The two step process is illustrated in Example 5 below.

The rifamycin derivatives

(c)   4-O-(n-butylsulphonyl)-3[(1-piperidinylimino) methyl]-rifamycin SV,

(d)   4-O-(n-butylsulphonyl)-3-[(4-morpholinylimino) methyl]-rifamycin SV and

(e)   3-[(dimethylhydrazono)methyl]-4-O-[(3-phenyl-propyl)sulphonyl]-rifamycin SV

may also be prepared in a manner described in said Japanese Kokai J5 6053-682 by reacting the appropriate 3-iminomethyl-rifamycin S derivative with an n-butylsulphonate or 3-phenylpropylsulphinate alkali metal or alkali earth metal salt under neutral conditions.

The precursors for (c), (d) and (e) above may be made by reacting (piperidinoimino)methane, (morpholino-imino)methane, or (dimethylaminoimino)methane respectively with rifamycin S as described in Japanese Kokai 79, 112,898, published September 4, 1979 (Chem. Abstracts no. 92:58837u). In this method the reaction may be carried out in the presence of an oxidizing agent such as manganese dioxide in dimethylsulphoxide under heating (50°C) for up to 30 hours. This method is also described in Japanese Kokai 80 89,286, published July 5, 1980 (Chemical Abstracts no. 94:65736e). Another method for obtaining these 3-iminomethyl rifamycin S derivatives is described in Japanese Kokai 78, 149,999, published December 27, 1978 (Chemical Abstracts no. 91:20555s).

Other methods for making the rifamycin derivatives (a) - (f) above are known as in the art, as are methods for interconverting rifamycin S and SV. For example, U.S. Patent no. 3,342,810, discloses a method in which 3-formyl-rifamycin SV is reacted with 1-aminopiperidine, 1-methylmorpholine or dimethylaminoamine to form the desired precursor 3-iminomethyl-rifamycin SV. The preparation of 3-formyl-rifamycin SV is also described therein. It is prepared from a selected Mannich base of rifamycin SV by oxidation with a weak oxidizing agent in a solvent. Such weak oxidizing agents include alkyl nitrates and lead tetracetate. Suitable Mannich bases may be selected from 3-pyrrolidinomethyl, 3-dimethylaminomethyl, 3-piperidinomethyl, 3-morpholinomethyl, 3-(2,6-dimethyl)piperidinomethyl, 3-(1-methyl)piperazinomethyl and 3-(4-carboxy) piperidinomethyl rifamycin SV, all of which are known in the art.

The ability of the rifamycin S derivatives (a) - (f) described above to inhibit the polymerase of hepatitis B virus was determined by the endogenous DNA polymerase assay according to Robinson and his colleagues as follows: In this procedure, the Dane particles were isolated from HBV-infected human blood by the method described by Landers et al., J. Virol 23:368-376, 1977.

- 11 -

Ten microliters of Dane particles were incubated at 37°C. for 2 hours in 100 µl containing 0.5% NP40, 0.07% BME, 66mM Tris HCl (pH 7.6) 27mMMbCl$_2$,0.40mM NH$_4$Cl,0.3mM, dATP, dGTP and dTTP and 4 picomoles $\alpha^{32}$p-dCTP. The appropriate tubes also contained either 0.1 µg/ml or 0.01 µg/ml of the rifamycin derivatives (a), (b), (c), (d), (e) or (f). The quantity of incorporation of radioactive nucletides into viral DNA was determined by trichloroacetic acid precipitation as described by Landers et al., J. Virol 23:368-376, 1977. The control DNA polymerase reaction contained more than 20,000 cpm. The results of this <u>in vitro</u> endogenous DNA polymerase assay for rifamycin derivatives (a) - (f) are given in Table I below:

<u>Table 1</u>

<u>In Vitro Anti-viral Polymerase Activity</u>

| Compound | %inhibition (0.1 µg/ml) | inhibition (0.01 µg/ml) |
|---|---|---|
| (a) | 54.1 | 17.2 |
| (b) | 89.0 | 10.0 |
| (c) | 55.7 | 8.3 |
| (d) | 92.5 | 10.2 |
| (e) | 85.6 | 25.1 |
| (f) | 84.6 | 23.8 |

As shown in Table I, the rifamycin derivatives (a-f) inhibited the Dane particle polymerase activity ranging from 54% to 92.5% at 0.1 µg/ml. Since Summers and Mason, <u>Cell</u>, above showed that the polymerase associated with the immature viral particles exhibits reverse transcriptase-like activity and Toh et al., <u>Nature</u>, above, showed a sub-stantial homology between HBV and WHV polymerase and 3 other retroviral reverse transcriptases, these DNA polymerase inhibition results are consistent with an inhibitory effect on the replication of the Dane particle by the polymerase.

Clinically (in man) persistance of serum marker of viral replication, such as HBeAg, HBV-DNA and DNA polymerase activity, is usually accompanied by persistance of chronic hepatitis disease activity, and conversely, disappearance of these markers is usually followed by a remission in the chronic hepatitis. Based upon serological testing, the course of chronic hepatitis B viral infection can be categorized in three outcomes (1) persistence of HBeAg (hepatitis Be antigen), HBV-DNA and DNA polymerase; (2) loss of HBeAg, HBV-DNA and DNA polymerase and seroconversion to anti-HBe; and (3) loss of HBeAg and seroconversion to anti-HBe but a subsequent reappearance of markers of HBV replication. Where (1), persistence of HBeAg, is found, persistence of the other markers of viral replication and of chronic hepatitis disease activity (plus elevated serum aminotransferase levels (ALT) is found. Where (2), seroconversion to anti-HBe, is found, the markers for viral replication are negative, serum ALT levels are normal and the disease is in remission. However, most of these subjects remain positive for HBsAg and are designated as "healthy" HBsAg carriers. Where (3), reactivation (known as spontaneous reactivation of hepatitis B virus), is found, intermittent symptoms of chronic hepatitis appear, during which increased ALT levels and the reappearance of HBV-DNA and DNA polymerase are observed. HBeAg may also reappear during reactivation. Episodes of reactivation can be severe and can mimic acute viral hepatitis. Subjects in whom such reactivation is seen tend to be older and to have had chronic type B hepatitis for a longer period of time. (See Hoofnagle and Alter, "Chronic Viral Hepatitis" Chapter 7, pp. 97-114, part. pp 98-102, in Vyas et al., eds., above.)

The diagnosis of chronic type B hepatitis is made by the finding of chronic elevations in serum aminotransferase levels (ALT) and persistence of HBeAg in the blood for at least 6 months. Most human subjects with active liver disease will also be positive for HBeAg. This serological marker is invariably present at the onset of the chronic HBeAg carrier state. With time, HBeAg may disappear and be replaced by anti-HBe. This seroconversion is usually, but not always, accompanied by a sustained decrease in the activity of the chronic liver disease.

Those subjects with anti-HBe who have ongoing chronic liver disease typically have other serological evidence of active viral replication, either low and intermittent levels of HBV-DNA and DNA polymerase or hepatitis B core antigen in liver. Another serological marker for the presence of active HBV replication and accompanying chronic hepatitis disease activity is IgM anti-HBc. This antibody may be present when HBV-DNA, DNA polymerase and HBeAg are no longer detectable or are present in only low titre. See Hoofnagle and Alter, above, pp. 103-4.

Hoofnagle and Alter, above, at page 100, report that liver biopsies taken on 85 human subjects who were HBsAg positive for at least six months (but not yet positive for anti-HBe) showed chronic persistent hepatitis in 26 per cent, chronic active hepatitis in 39 per cent and chronic active hepatitis with cirrhosis in 20 per cent, respectively, of said subjects.

An animal, particularly a primate such as man, infected with hepatitis B virus may preferably receive one of two dose regimens.

Said subject may receive either (1) daily injection of the compound at a dose range of 1 to 20 mg/kg (body weight) for a period of 2 weeks or (2) daily oral administration of 10 to 50 mg/kg for one month (or for longer periods as determined by the subject's physician). To follow the state of disease, subjects may be monitored with virological markers such as DNA polymerase (PNAS 77 2941 [1980]) as well as HBsAg, HBeAg, antibody to HBsAg (Anti-HBs), and antibody to HBeAg (Anti-HBe) (J. Infectious Diseases 148, 907 [1983]). The polymerase levels may be determined twice a week during the period. The other assays would be performed weekly. Serum glutamic oxaloacetic transaminase levels (SCOT) could also be monitored weekly. Alternatively, serum alanine aminotransferase (ALT) and aspartate aminotransferase (AST) levels could also be monitored. The frequency and length of the drug administration and monitoring could be adjusted according to information obtained from the levels of virological markers.

Such course of treatment could be used in combination with other appropriate therapies.

The rifamycin derivatives a-f can be administered in the form of a composition comprising the active ingredient in association with a pharmaceutically acceptable carrier. Any suitable carrier known in the art can be used to prepare the pharmaceutical composition. In such a composition, the carrier is generally a solid or liquid or a mixture of a solid and a liquid.

Solid form compositions include powders, granules, tablets, capsules (e.g. hard and soft gelatine capsules), suppositories and pessaries. A solid carrier can be, for example, one or more substances which may also act as flavouring agents, lubricants, solubilisers, suspending agents, fillers, glidants, compression aides, binders or tablet-disintegrating agents; it can also be an encapsulating material. In powers the carrier is a finely divided solid which is in admixture with the finely divided active ingredient. In tablets the active ingredient is mixed with a carrier having the necessary compression properties in suitable proportions and compacted in the shape and size desired. The powders and tablets preferably contain up to 99%, e.g. from 0.03 to 99%, preferably 1 to 80% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, poly-vinylpyrrolidine, low melting waxes and ion exchange resins.

The term "composition" is intended to include the formulation of an active ingredient with encapsulating material as carrier to give a capsule in which the active ingredient (with or without other carriers) is surrounded by the carrier, which is thus in association with it. Similarly cachets are included.

Liquid form compositions include, for example, solutions, suspensions, emulsions, syrups, elixirs and pressurised compositions. The active ingredient, for example, can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavouring agents, suspending agents, thickening agents, colours, viscosity regulators, stabilisers or osmo-regulators. Suitable examples of liquid carriers for oral and parenteral administration include water (particularly containing additives as above e.g. cellulose derivatives, preferably sodium carboxymethyl cellulose solution), alcohols (including monohydric alcohols and polyhydric alcohols e.g. glycerol and glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intraveneously. When the compound is orally active it can be administered orally either in liquid or solid composition form.

Preferably the pharmaceutical composition is in unit dosage form, e.g. as tablets or capsules. In such form, the composition is sub-divided in unit dose containing appropriate quantities of the active ingredient; the unit dosage can be packaged composition, for example packed powders, vials, ampoules, prefilled syringes or sachets containing liquids. The unit dosage form can be, for example, a capsule or tablet itself, or it can be the appropriate number of any such compositions in package form.

- 16 -

The following Examples 1 to 5 illustrate the preparation of the novel rifamycin derivatives of the present invention

## Example 1

3-Amino-4-deoxo-4-[(4-aminophenyl)sulphonyl]aminorifamycin SV (b) :

A solution of 2.6g of sodium 4-aminobenzensulphinate in 5 ml of water was added to a solution of 2.8g of 3-amino-4-deoxo-4-iminorifamycin S in 50 ml of dimethyl sulphoxide. The mixture was stirred at toom temperature for 5 min and then poured into brine. After acidification with dil.$H_2SO_4$ the reaction mixture was extracted with chloroform. The extract was washed with brine, dried over anhydrous sodium sulphate, and then evaporated. The residue was precipitated from chloroform hexane to give 2.2g of 3-amino-4-deoxo-4-[(4-aminophenyl)sulphonyl]amino-rifamycin SV as a yellow powder.

IR ($CHCl_3$) : $1156cm^{-1}$ (sulphonamide).

The nmr spectrum ($CDCl_3$) shows the characteristic singlet signals of the methyl groups at $\delta 1.59$(3H), 2.08 (6H), 2.14(3H) and 3.02(3H) ppm.

## Example 2

4-O-Butylsulphonyl-3-(piperidinoimino)methylrifamycin SV (c) :

A solution of 2.0g of sodium butylsulphinate in 10ml of water was added to a solution of 2.5g of 3-(piperid-inoimino)methylrifamycin S in 100ml of N,N-dimethylformamide. The mixture was stirred at room temperature for 9 hr and then poured into brine. After acidification with dil.$H_2SO_4$ the reaction mixture was extracted with ethyl acetate. The extract was washed with brine, dried over anhydrous sodium sulphate, and then evaporated to dryness. The residue was subjected to silica gel column chromatography [solvent: chloroform-methanol (20.1)] and then recrystallized from ethyl acetatehexane to give 1.5g of 4-O-butylsulphonyl-3-(piperidinoimino)methylrifamycin SV as yellow crystals

IR (KBr) : 1165cm$^{-1}$ (sulphonate).

The nmr spectrum (CDCl$_3$) shows the characteristic singlet signals of the hydrazonomethyl group at δ8.02(1H) and those of the methyl groups at δ1.76(3H), 2.06(3H), 2.10(3H), 2.28(3H) and 3.6(3H) ppm.


Example 3


4-O-Butylsulphonyl-3-(morpholinoimino)methylrifamycin SV (d) :


A solution of 2.0g of sodium butylsulphinate in 10 ml of water was added to a solution of 3.0g of 3-(morpholinoimino)methylrifamycin S in 150ml of dimethyl sulphoxide. The mixture was stirred at room temperature for 20 hr and then poured into brine. After acidification with dil.H$_2$SO$_4$, the reaction mixture was extracted with ethyl acetate. The extract was washed with brine over anhydrous sodium sulphate, then evaporated to dryness. The residue was subjected to silica gel column chromatography [solvent; chloroform-methanol (10.1)], and then recrystallized from ethylacetate-hexane to give 1.08g of 4-O-butylsulphonyl-3-(morpholinoimino)methylrifamycin SV as yellow crystals.

IR (KBr) : 1165cm$^{-1}$ (sulphonate).

The nmr spectrum (CDCl$_3$) shows the characteristic singlet signals of the hydrazonomethyl group at δ8.16(1H) and those of the methyl groups at δ1.78(3H), 2.07(3H), 2.12 (3H), 2.30(3H) and 3.08(3H) ppm.


Example 4


3-[(Dimethylhydrazono)methyl]-4-O-(3-phenylpropyl)sulphonylrifamycin SV (e) :


A solution of 3.0g of sodium 3-phenylpropane sulphinate in 15ml of water was added to a solution of 3.0g of 3-(dimethylhydrazono)methylrifamycin S in 150 ml of dimethyl sulphoxide. The mixture was stirred at room temperature for 24 hr and then poured into brine. After acidification with dil.$H_2SO_4$, the reaction mixture was extracted with ethyl acetate. The extract was washed with brine, dried over anhydrous sodium sulphate, and then evaporated to dryness. The residue was twice subjected to silica gel column chromatography [solvent:chloroform-methanol (85.1), chloroformacetone (4.1), respectively], and then recrystallized from ethylacetate-ligroin to give 0.7g of 3-[(dimethylhydrazono)methyl]-4-O-(3-phenylpropyl)-sulphonylrifamycin SV as yellow crystals.

IR (KBr) : 1160cm$^{-1}$ (sulphonate).

The nmr spectrum (CDCl$_3$) shows the characteristic singlet signals of the hydrazonomethyl group at δ7.66(1H) and those of the methyl groups at δ1.68(3H), 2.05(3H), 2.09 (3H), 2.28(3H), 2.95(6H) and 3.03(3H) ppm.

## Example 5

### 4-Deoxy-N,3-ethylidenimino-4-(methylsulphonyl)aminorifamycin SV (f) :

(1)  3-Amino-4-deoxy-4-(methylsulphonyl)aminorifamycin SV:

A solution of 2.3g of sodium methanesulphinate in 15ml of water was added to a solution of 5.3g of 3-amino-4-deoxy-4-iminorifamycin S in 90ml of dimethyl sulphoxide. The mixture was stirred at room temperature for 10 min and then poured into brine. After acidification with dil.$H_2SO_4$, the reaction mixture was extracted with chloroform. The extract was washed with brine, dried over anhydrous sodium sulphate, and then evaporated. The residue was precipitated from chloroform hexane to give 3.9g of 3-amino-4-deoxy-4-(methylsulphonyl)aminorifamycin SV as a yellow powder.

IR(CHCl$_3$) : 1155cm$^{-1}$ (sulphonamide).

The nmr spectrum (CDCl$_3$) shows the characteristic singlet signals of the methyl groups at δ1.80(3H), 2.08(6H), 2.24 (3H), 2.63(3H) and 3.07(3H).

(2)  4 Deoxy-N,3-ethylidenimino-4-(methylsulphonyl)amino-rifamycin SV:

Two ml of acetaldehyde was added to a solution of 3.0g of 3-amino-4-deoxy-4-(methylsulphonyl)aminorifamycin SV in 100ml of tetrahydrofuran. The mixture was stirred at room temperature for 4 hr, and then evaporated to dryness. The residue was subjected to silica gel column chromatography [solvent; chloroform-methanol (50:1)], and then precipitated from chloroform-hexane to give 1.5g of 4-deoxy-N,3-ethylidenimino-4-(methylsulphonyl)amino-rifamycin SV as a yellow powder.

IR (CDCl$_3$) : 1147cm$^{-1}$ (sulphonamide). The nmr spectrum (CDCl$_3$) shows the characteristic doublet signal of the methyl group of the ethylidenimino group at $\delta$1.49(3H) and the characteristic singlet signals of the methyl groups at $\delta$1.78(3H),2.09(9H), 2.69(3H) and 3.11(3H).

AHP-8615-EPC

0213935

- 20 -

CLAIMS

1.   The use of a rifamycin derivative selected from the
group consisting of

    (a)   3-amino-4-deoxo-4-imino-rifamycin S,
    (b)   3-amino-4-deoxo-4-[(4-aminophenyl)sulphonyl]-
          amino-rifamycin SV,
    (c)   4-O-n-butylsulphonyl-3-[(1-piperidinylimino)-
          methyl]-rifamycin SV,
    (d)   4-O-n-butylsulphonyl-3-[(4-morpholinylimino)-
          methyl]-rifamycin SV,
    (e)   3-[(dimethylhydrazono)methyl]-4-O-[(3-phenylpropyl)-sulphonyl]-rifamycin SV and
    (f)   4-deoxy-N,3-ethylidenimino-4-(methylsulphonyl)-
          amino-rifamycin SV

for the manufacture of a medicament for the treatment of an
animal infected by hepatitis B virus or for the manufacture
of a medicament for the prevention of hepatitis in an
animal suspected of having been infected with hepatitis
B virus.

2.   The use as claimed in Claim 1 wherein the rifamycin
derivative is 3-[(dimethylhydrazono)methyl]-4-O-[(3-
phenylpropyl)sulphonyl]-rifamycin SV.

3.   The use as claimed in Claim 1 wherein the rifamycin
derivative is 4-deoxy-N,3-ethylidenimino-4-(methylsulphonyl)amino-rifamycin SV.

4.   A rifamycin derivative selected from a group consisting
of

    (b)   3-amino-4-deoxo-4-[(4-aminophenyl)sulphonyl]-
          amino-rifamycin SV,
    (c)   4-O-n-butylsulphonyl-3-[(1-piperidinylimino)-
          methyl]-rifamycin SV,
    (d)   4-O-n-butylsulphonyl-3-[(4-morpholinylimino)-
          methyl]-rifamycin SV,
    (e)   3-[(dimethylhydrazono)methyl]-4-O-[(3-phenylpropyl)sulphonyl]-rifamycin SV and

(f)    4-deoxy-N,3-ethylidenimino-4-(methylsulphonyl)-
       amino-rifamycin SV.

5.    3-[(Dimethylhydrazono)methyl]-4-O-[(3-phenylpropyl)-
sulphonyl]-rifamycin SV.

6.    4-Deoxy-N,3-ethylidenimino-4-(methylsulphonyl)amino-
rifamycin SV.

CLAIMS (FOR AUSTRIA)

1. The use of a rifamycin derivative selected from the group consisting of

    (a)    3-amino-4-deoxo-4-imino-rifamycin S,

    (b)    3-amino-4-deoxo-4-[(4-aminophenyl)sulphonyl]-amino-rifamycin SV,

    (c)    4-O-n-butylsulphonyl-3-[(1-piperidinylimino)-methyl]-rifamycin SV,

    (d)    4-O-n-butylsulphonyl-3-[(4-morpholinylimino)-methyl]-rifamycin SV,

    (e)    3-[(dimethylhydrazono)methyl]-4-O-[(3-phenylpropyl)sulphonyl]-rifamycin SV and

    (f)    4-deoxy-N,3-ethylidenimino-4-(methylsulphonyl)-amino-rifamycin SV

for the manufacture of a medicament for the treatment of an animal infected by hepatitis B virus or for the manufacture of a medicament for the prevention of hepatitis in an animal suspected of having been infected with hepatitis B virus.

2. The use as claimed in Claim 1 wherein the rifamycin derivative is 3-[(dimethylhydrazono)methyl]-4-O-[(3-phenylpropyl)sulphonyl]-rifamycin SV.

3. The use as claimed in Claim 1 wherein the rifamycin derivative is 4-deoxy-N,3-ethylidenimino-4-(methylsulphonyl)amino-rifamycin SV.

4. A process for preparing 3-amino-4-deoxo-4-[(4-aminophenyl)sulphonyl]amino-rifamycin SV which comprises reacting an alkali metal p-analinylsulphinate with 3-amino-rifamycin S.

5. A process for preparing 3-amino-4-deoxo-4-[(4-aminophenyl)sulphonyl]amino-rifamycin SV which comprises reacting an alkali metal 4-aminobenzenesulphinate with 3-amino-4-deoxo-4-iminorifamycin S.

6. A process for preparing 4-O-n-butylsulphonyl-3-[(1-piperidinylimino)methyl]-rifamycin SV, 4-O-n-butylsulphonyl-3-[(4-morpholinylimino)methyl]-rifamycin SV or 3-[(dimethylhydrazono)methyl]-4-O-[(3-phenylpropyl)-sulphonyl]-rifamycin SV which comprises reacting an appropriate 3-iminomethyl-rifamycin S derivative with an n-butylsulphonate or 3-phenylpropylsulphinate alkali metal or alkali earth metal salt.

7. A process for preparing 4-O-n-butylsulphonyl-3-[(1-piperidinylimino)methyl]-rifamycin SV which comprises reacting an alkali metal butylsulphinate with 3-(piperidino-imino)methylrifamycin S.

8. A process for preparing 4-O-n-butylsulphonyl-3-[(4-morpholinylimino)methyl]-rifamycin SV which comprises reacting an alkali metal butylsulphinate with 3-(morpholino-imino)methylrifamycin S.

9. A process for preparing 3-[(dimethylhydrazono)methyl]-4-O-[(3-phenylpropyl)sulphonyl]-rifamycin SV which comprises reacting an alkali metal 3-phenylpropanesulphinate with 3-(dimethylhydrazono)methylrifamycin S.

10. A process for preparing 4-deoxy-N,3-ethylidenimino-4-(methylsulphonyl)amino-rifamycin SV which comprises reacting acetaldehyde with 3-amino-4-deoxy-4-(methyl-sulphonyl)amino-rifamycin SV.